# EUROPEAN PATENT APPLICATION

(11) **EP 4 289 468 A1**
(43) Date of publication of application: **13.12.2023**
(21) Application number: 22762382.4
(22) Date of filing: 18.02.2022
(51) Int. Cl.: A61N 1/36

(54) **ELECTRICAL STIMULATION HAIR GROWTH APPARATUS**

(30) Priority: 04.03.2021 CN 202110238030; 04.03.2021 CN 202120464858 U
(71) Applicant: Nazhiyuan Technology (Tangshan), LLC, Tangshan, Hebei 063000 (CN)
(72) Inventor: XU, Chuanyi, Tangshan, Hebei 063000 (CN)
(74) Representative: Chung, Hoi Kan
(86) International application number: PCT/CN2022/076834
(87) International publication number: WO 2022/183916

(57) **Abstract**

The present disclosure provides an electrical stimulation hair growing device, comprising: a vibration module, an electrical pulse module and an electrical stimulation module; wherein: the vibration module is used for generating vibration with a predetermined frequency and a predetermined amplitude; the electrical pulse module is provided in contact with the vibration module, and is used for converting vibration acted thereon by the vibration module into electrical pulses and outputting the electrical pulses, and/or for converting mechanical energy acted thereon by external environment into electrical pulses and outputting the electrical pulses; and the electrical stimulation module is provided on an area in need of hair growing, is connected to the electrical pulse module and is used for acting the electrical pulses output by the electrical pulse module on the area in need of hair growing for electrical stimulation.

## Description

### CROSS-REFERENCE TO RELATED APPLICATIONS

The present application claims priority of a Chinese patent application filed with China Patent Office on March 04, 2021 with an application number of 202110238030.6 and an application title of "Electrical Stimulation Hair Growing Device", and a Chinese patent application filed with China Patent Office on March 04, 2021 with an application number of 202120464858.9 and an application title of "Electrical Stimulation Hair Growing Device", and the contents thereof are entirely incorporated here by reference.

### TECHNICAL FIELD

The present disclosure relates to the technical field of biomedicine, in particular to an electrical stimulation hair growing device.

### BACKGROUND

In recent years, hair loss has gradually turned into a topic of concern. As shown by the latest data from the National Health Commission of the People's Republic of China, more than 250 million people in China have the problem of hair loss, i.e., an average of 1 in less than 6 people. Hair loss happens for various reasons, such as long-term extreme fatigue and anxiety under excessive mental stress, or congenital genetic factors. Hair loss will not cause serious damages to human body or endanger human lives, but it will seriously impact all aspects of people's daily life, such as job hunting, marriage and so on. Therefore, it is urgent in modern society to treat hair loss.

Currently, there are various ways of hair growing, such as drug hair growing, acupuncture hair growing and electrical stimulation hair growing, among which electrical stimulation hair growing is widely accepted and used as a simple, harmless, reliable and practical means of hair growing. Although a lot of improvements have been made to reduce the size and weight of traditional electrical stimulation hair growing devices, they are still bulky and non-portable, and cannot be used without external power supply, as a result, the use thereof by users is badly limited. In order to solve the above problem, an electrical stimulation hair growing device based on triboelectricity generating technology emerged.

Although the electrical stimulation hair growing devices based on triboelectricity generating technology in the prior art can be used without external power supply, they cannot accurately apply different electrical pulses for electrically stimulating hair growing specific to depilation or hair loss conditions of different objects in need of hair growing.

Therefore, in the prior art, an electrical stimulation hair growing device, which is capable of accurately applying different electrical pulses for electrically stimulating hair growing specific to depilation or hair loss conditions of different objects in need of hair growing, is lacking.

### SUMMARY

As for the afore-mentioned problem, the present disclosure discloses an electrical stimulation hair growing device, so as to solve or at least partly solve the afore-mentioned problem.

In order to realize the afore-mentioned object, the technical solution below is adopted in the present disclosure.

The present disclosure provides an electrical stimulation hair growing device, comprising: a vibration module, an electrical pulse module and an electrical stimulation module; wherein: the vibration module is used for generating vibration with a predetermined frequency and a predetermined amplitude; the electrical pulse module is provided in contact with the vibration module, and is used for converting vibration acted thereon by the vibration module into electrical pulses and outputting the electrical pulses, and/or for converting mechanical energy acted thereon by external environment into electrical pulses and outputting the electrical pulses; and the electrical stimulation module is provided on an area in need of hair growing, is connected to the electrical pulse module and is used for acting the electrical pulses output by the electrical pulse module on the area in need of hair growing for electrical stimulation.

Further, the vibration module further comprises: a power module, a driver module, and at least one vibration part; wherein: the power module is connected to the driver module, and is used for providing electric energy to the driver module; the driver module is connected to the at least one vibration part, and is used for controlling the at least one vibration part to vibrate at the predetermined frequency and the predetermined amplitude; and the at least one vibration part is provided in contact with the electrical pulse module, and is used for generating vibration having the predetermined frequency and the predetermined amplitude so as to act the vibration on the electrical pulse module.

Further, the power module further comprises a battery, a vibration switch module and a power management module; wherein: the battery is connected to the vibration switch module, and is used for providing electric energy to the power management module through the vibration switch module; the vibration switch module is connected to the power management module, and is used for controlling whether the battery provides electric energy to the power management module; and the power management module is connected to the driver module, and is used for converting the electric energy output by the battery through the vibration switch module, so as to provide electric energy to the driver module.

Further, the electrical stimulation hair growing device further comprises an electrical stimulation switch module; wherein: the electrical stimulation switch module is respectively connected to the electrical pulse module and the electrical stimulation module, and is used for enabling the electrical pulse module to output electrical pulses to the electrical stimulation module when the electrical pulse module and the electrical stimulation module are conducted by the electrical stimulation switch module.

Further, the electrical stimulation hair growing device further comprises an electrical stimulation switch module and a charging module; wherein: the electrical stimulation switch module is respectively connected to the electrical pulse module and the electrical stimulation module, and is used for enabling the electrical pulse module to output electrical pulses to the electrical stimulation module when the electrical pulse module and the electrical stimulation module are conducted by the electrical stimulation switch module; the charging module is respectively connected to the electrical stimulation switch module and the power module, and is used for preprocessing the electrical pulses output by the electrical pulse module when the electrical pulse module and the charging module are conducted by the electrical stimulation switch module, and for outputting the preprocessed electrical pulses to the power module for charging.

Further, a transmission insulating part for transmitting vibration and for avoiding conduction of the at least one vibration part and the electrical pulse module is further provided between the at least one vibration part and the electrical pulse module.

Further, the vibration part is a rotor motor, a longitudinal linear motor or a transverse linear motor.

Further, the number of vibration parts is multiple, wherein: the multiple vibration parts comprise the same number of the longitudinal linear motors and the transverse linear motors, and the longitudinal linear motors and the transverse linear motors are uniformly distributed in arrays on the electrical pulse module.

Further, the electrical stimulation module further comprises a plurality of electrodes; and at least one electrical stimulation bulge is provided on a side surface where the plurality of electrodes is in contact with the area in need of hair growing.

Further, the electrical pulse module comprises: a triboelectric generator, and/or a piezoelectric generator, and/or a pressure inductive cable based on a triboelectric generator and/or a piezoelectric generator, and/or a piezoelectric and triboelectric hybrid generator.

Further, the triboelectric generator is a triboelectricity generating film; and/or the piezoelectric generator is a piezoelectricity generating film.

Further, the triboelectric generator comprises a friction part-based knitted triboelectric generator; wherein: the friction part-based knitted triboelectric generator comprises: at least one first friction part and at least one second friction part; wherein: the at least one first friction part and the at least one second friction part are knitted with each other to form the friction part-based knitted triboelectric generator, and at least one of the at least one first friction part and the at least one second friction part comprises a friction interface capable of contacting friction; and the at least one first friction part and/or the at least one second friction part have an electrical signal output end of the friction part-based knitted triboelectric generator.

Further, the pressure inductive cable based on a triboelectric generator and/or a piezoelectric generator is knitted to form the electrical pulse module having a predetermined shape.

Further, the at least one first friction part and the at least one second friction part are knitted to form the electrical pulse module having a predetermined shape.

Further, the predetermined shape is a hat or cap shape, a headgear shape, a turban shape, a collar shape or a sheet shape.

Further, the electrical stimulation hair growing device further comprises a wearable body; the wearable body is worn on an object in needed of hair growing, and is provided with the at least one vibration module, the electrical pulse module and/or the electrical stimulation module.

Further, the wearable body is a hat or cap, a headgear, a turban, a collar or a sheet.

Further, the electrical stimulation hair growing device is a detachable and/or portable electrical stimulation hair growing device.

Following are advantages and beneficial effects of the present disclosure.
(1) The electrical stimulation hair growing device provided in the present disclosure generates vibration with a predetermined frequency and a predetermined amplitude through the vibration module, and accurately controls the electrical pulses output by the electrical pulse module to the electrical stimulation module, so as to accurately apply different electrical pulses to different objects in need of hair growing, thereby activating epidermal hair follicles and promoting hair growth.
(2) Since a triboelectric generator, and/or a piezoelectric generator, and/or a pressure inductive cable based on a triboelectric generator and/or a piezoelectric generator, and/or a piezoelectric and triboelectric hybrid generator are adopted in the electrical pulse module, the electrical stimulation hair growing device provided in the present disclosure can directly convert the mechanical energy in external environment into electrical pulses when there is no or insufficient external power supply, so that electrical stimulation can be performed by the electrical stimulation module, which is energy-efficient and environmentally friendly, and can also avoid the failure to use the electrical stimulation hair growing device caused by the absence of external power supply.
(3) The electrical stimulation hair growing device provided in the present disclosure can convert the mechanical energy in external environment into electric energy by means of the electrical pulse module, so as to charge the power module, which can prolong the power supply time of the power module, as well as is energy-efficient and environmentally friendly.
(4) Since a triboelectric generator, and/or a piezoelectric generator, and/or a pressure inductive cable based on a triboelectric generator and/or a piezoelectric generator, and/or a piezoelectric and triboelectric hybrid generator are adopted in the electrical pulse module, the electrical stimulation hair growing device provided in the present disclosure is light and small, and is easy for users to carry and/or use.
(5) The electrical stimulation hair growing device provided in the present disclosure is simple in structure and manufacturing process, and has low cost, so it is suitable for large-scale industrial production.

### BRIEF DESCRIPTION OF DRAWINGS

All other advantages and beneficial effects will be clear and apparent for ordinary technicians in this field after reading detailed description of the preferable embodiments below. The drawings are included only to show the preferable embodiments, and should not be regarded as limits of the present disclosure. Besides, a single reference symbol represents the same part in all drawings. In the drawings:
FIG. 1 is a structure diagram showing the electrical stimulation hair growing device in one example of the present disclosure;
FIG. 2 is a diagram showing a circuit module of the vibration module in one example of the present disclosure;
FIG. 3 is a diagram showing another circuit module of the vibration module in one example of the present disclosure;
FIG. 4 is a structure diagram showing at least one first friction part of the friction part-based knitted triboelectric generator in one example of the present disclosure;
FIG. 5 is another structure diagram showing at least one first friction part of the friction part-based knitted triboelectric generator in one example of the present disclosure;
FIG. 6 is a structure diagram showing an electrode of the electrical stimulation module in an example of the present disclosure;
FIG. 7 is another structure diagram showing the electrical stimulation hair growing device in one example of the present disclosure; and
FIG. 8 is a diagram showing a circuit module of the electrical stimulation hair growing device in another example of the present disclosure.

### DETAILED DESCRIPTION OF THE EMBODIMENTS

In order to make the object, the technical solution and the advantages of the present disclosure clearer, the technical solution of the present disclosure will be clearly and completely illustrated with reference to examples and corresponding drawings of the present disclosure. Obviously, the examples illustrated are merely part of the examples of the present disclosure, rather than all. Based on the examples of the present disclosure, all other examples obtained by one skilled in the art without paying creative efforts belong to the scope of protection of the present disclosure.

With reference to the drawings, technical solutions in all examples of the present disclosure will be illustrated in detail hereinafter.

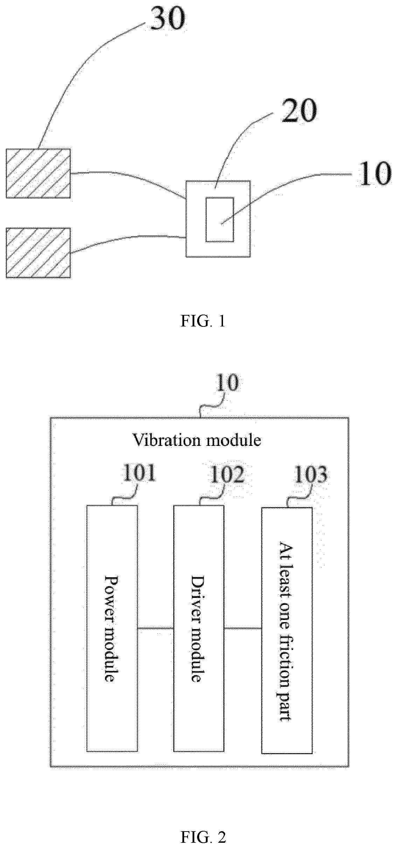

FIG. 1 is a structure diagram showing Example 1 of the electrical stimulation hair growing device provided in the present disclosure. As shown in FIG. 1, the electrical stimulation hair growing device of Example 1 comprises: a vibration module 10, an electrical pulse module 20 and an electrical stimulation module 30; wherein: the vibration module 10 is used for generating vibration with a predetermined frequency and a predetermined amplitude; the electrical pulse module 20 is provided in contact with the vibration module 10, and is used for converting vibration acted thereon by the vibration module 10 into electrical pulses and outputting the electrical pulses, and/or for converting mechanical energy acted thereon by external environment into electrical pulses and outputting the electrical pulses; and the electrical stimulation module 30 is provided on an area in need of hair growing (not shown), is connected to the electrical pulse module 20 and is used for acting the electrical pulses output by the electrical pulse module 20 on the area in need of hair growing for electrical stimulation.

Further, as shown in Figs. 1 and 2, the vibration module 10 may comprise: a power module 101, a driver module 102, and at least one vibration part 103; wherein: the power module 101 is connected to the driver module 102, and is used for providing electric energy to the driver module 102; the driver module 102 is connected to the at least one vibration part 103, and is used for controlling the at least one vibration part 103 to vibrate at the predetermined frequency and the predetermined amplitude; and the at least one vibration part 103 is provided in contact with the electrical pulse module 20, and is used for generating vibration having the predetermined frequency and the predetermined amplitude so as to act the vibration on the electrical pulse module 20.

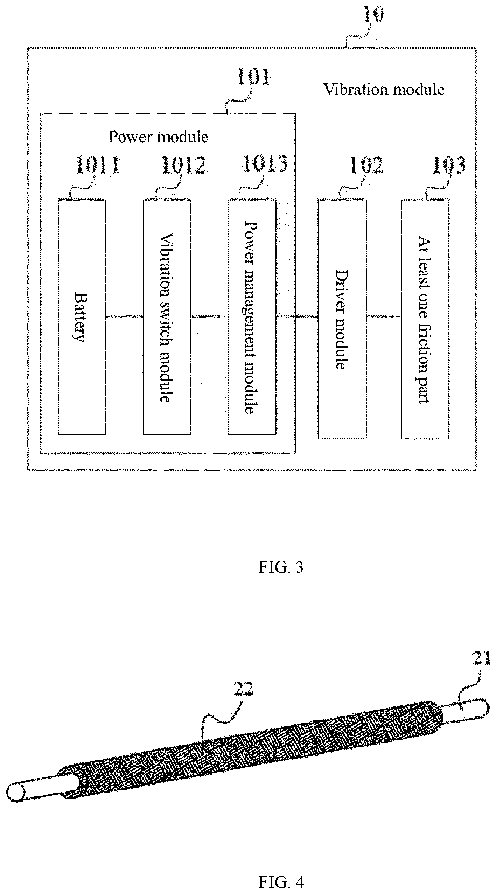

Optionally, as shown in Figs. 1 and 3, the power module 101 further comprises a battery 1011, a vibration switch module 1012 and a power management module 1013; wherein: the battery 1011 is connected to the vibration switch module 1012, and is used for providing electric energy to the power management module 1013 through the vibration switch module 1012; the vibration switch module 1012 is connected to the power management module 1013, and is used for controlling whether the battery 1011 provides electric energy to the power management module 1013; and the power management module 1013 is connected to the driver module 102, and is used for converting the electric energy output by the battery 1011 through the vibration switch module 1012, so as to provide electric energy to the driver module 20.

In Example 1, the battery 1011 can be a battery in the prior art, and the type thereof can be selected by one skilled in the art according to actual necessities and will not be limited here. For example, the battery 1011 can be a rechargeable or non-rechargeable nickel-cadmium battery, nickel-metal hydride battery, lithium-ion battery, or lead storage battery.

The vibration switch module 1012 can be a switch in the prior art. For example, the vibration switch module 1012 can be a key switch, a touch switch or a film switch, and the type thereof can be selected by one skilled in the art according to actual necessities and will not be limited here. Since film switches are light, thin and flexible, which can increase the comfort of use, the vibration switch module 1012 is preferably a film switch.

Specifically, when the vibration switch module 1012 is turned on, the vibration switch module 1012 conducts the battery 1011 and the power management module 1013, and the battery 1011 provides electric energy to the power management module 1013 through the vibration switch module 1012; when the vibration switch module 1012 is turned off, the vibration switch module 1012 disconnects the battery 1011 from the power management module 1013, and the battery 1011 stops providing electric energy to the power management module 1013 through the vibration switch module 1012.

The function of the power management module 1013 is to convert the electric energy output by the battery 1011 through the vibration switch module 1012 into a voltage suitable for the driver module 102. Therefore, the power management module 1013 can be a power management chip in the prior art capable of converting the electric energy output by the battery 1011 through the vibration switch module 1012 into a voltage suitable for the driver module 102, which will not be limited here.

Wherein: the driver module 102 can be a driver chip in the prior art, and the type thereof can be selected by one skilled in the art according to actual necessities and will not be limited here.

Wherein: the at least one vibration part 103 is a vibration part in the prior art, and the type thereof can be selected by one skilled in the art according to actual necessities and will not be limited here. For example, the at least one vibration part 103 is preferably a rotor motor, a longitudinal linear motor or a transverse linear motor. In addition, the number of the at least one vibration part 103 may be one or more, which can be selected by one skilled in the art according to actual necessities and will not be limited here.

In a preferable embodiment, the number of vibration parts 103 is multiple, and the multiple vibration parts 103 comprise the same number of longitudinal linear motors and transverse linear motors, which are uniformly distributed in arrays on the electrical pulse module 20. This way of arrangement is to enable the multiple vibration parts 103 to apply longitudinal and transverse vibration to the electrical pulse module 20, so as to accurately control the predetermined frequency and predetermined amplitude of the electrical pulses output by the electrical pulse module 20.

It should be understood that one or more longitudinal linear motors can be provided in contact with the electrical pulse module 20 if it is required to apply only longitudinal vibration to the electrical pulse module 20 through the at least one vibration part 103, and one or more lateral linear motors can be provided in contact with the electrical pulse module 20 if it is required to apply only transverse vibration to the electrical pulse module 20 through the at least one vibration part 103.

In the present disclosure, as the number of the vibration parts 103 increases, the intensity (i.e., the amplitude) of the vibration applied to the electrical pulse module 20 also increases, namely, there are two ways of increasing intensity of the vibration applied to the electrical pulse module 20: one is to increase the vibration intensity applied to the electrical pulse module 20 through the driver module 102, and the other one is to increase the vibration intensity applied to the electrical pulse module 20 by providing a plurality of vibration parts 103. These two ways can be selected by one skilled in the art according to actual necessities, and will not be limited here.

Wherein: the providing position of the vibration module 10 can be selected by one skilled in the art according to actual necessities, and will not be limited here. However, it must be sure that the vibration generated by the vibration module 10 can be applied to the electrical pulse module 20.

In an optional embodiment, as shown in FIG. 1, the vibration module 10 is provided in contact with the electrical pulse module 20, so that the vibration generated by the vibration module 10 can be better applied to the electrical pulse module 20, wherein the vibration module 10 can be provided in direct or indirect contact with the electrical pulse module 20. In addition, the vibration module 10 is provided in indirect contact with the electrical pulse module 20 to avoid conduction of the vibration module 10 and the electrical pulse module 20. Therefore, a transmission insulating part (not shown), which is used for transmitting vibration and avoiding conduction of the vibration module 10 and the electrical pulse module 20, is further provided between the vibration module 10 and the electrical pulse module 20, wherein the transmission insulating part can be selected from insulating films in the prior art, such as polyvinyl chloride film, polyethylene film, polypropylene film, polystyrene film, etc.

Specifically, if the vibration module 10 comprises a power module 101, a driver module 102 and at least one vibration part 103, any or more of the power module 101, the driver module 102 and the at least one vibration part 103 can be provided in direct or indirect contact with the electrical pulse module 20; however, it must be sure that the vibration generated by the at least one vibration part 103 can be applied to the electrical pulse module 20. Preferably, the at least one vibration part 103 is provided in direct or indirect contact with the electrical pulse module 20; moreover, in order to avoid conduction of the at least one vibration part 103 and the electrical pulse module 20, a transmission insulating part (not shown), which is used for transmitting vibration and avoiding conduction of the at least one vibration part 103 and the electrical pulse module 20, is further provided between the at least one vibration part 103 and the electrical pulse module 20, wherein the transmission insulating part can be selected from insulating films in the prior art, such as polyvinyl chloride film, polyethylene film, polypropylene film, polystyrene film, etc.

In this example, as for the types of the power management module 1013, the driver module 102 and the at least one vibration part 103, one skilled in the art can select the power management chip, the driver chip and the vibration part in the prior art according to actual necessities, which will not be limited here. However, it should be noted that, when selecting types of the power management module 1013, the driver module 102 and the at least one vibration part 103, one skilled in the art should ensure that parameters of the three match with each other. For example, when the driving voltage of the driver module 102 is a DC voltage of 3.3 V, it is necessary that the power management module 1013 can output a DC voltage of 3.3 V. Similarly, when the starting voltage of the at least one vibration part 103 is a DC voltage of 2.3 V, it is necessary that the driver module 102 can output a DC voltage of 2.3 V.

The electrical pulse module 20 in Example 1 can be provided on an object in need of hair growing, such as a human body, an animal body and other organisms that need hair growing. When vibration is generated by the vibration module 10 or the mechanical energy from external environment is acted on the object in need of hair growing, reciprocating extrusion or loosening occurs between the electrical pulse module 20 and the object in need of hair growing, and relative motion is generated due to relative friction, and then is converted into electrical pulses and is output. However, it should be understood that the electrical pulse module 20 can also be provided on other objects than the object in need of hair growing. For example, the electrical pulse module 20 can be provided on a table, and vibration can be applied by the vibration module 10 or by hand tapping or the like, so that the electrical pulse module 20 generates electrical pulses.

Further, the electrical pulse module 20 may comprise a triboelectric generator, and/or a piezoelectric generator, and/or a pressure inductive cable based on a triboelectric generator and/or a piezoelectric generator, and/or a piezoelectric and triboelectric hybrid generator in the prior art. The type of the electrical pulse module 20 can be selected by one skilled in the art according to actual necessities, and will not be limited here.

Wherein: the triboelectric generator can be one in the prior art, which has a three-layer structure, a four-layer structure, a five-layer intermediate film structure or a five-layer intermediate electrode structure; the above-mentioned triboelectric generator comprises at least two surfaces constituting friction interfaces, and has an output end; the piezoelectric generator can be one in the prior art, which is made of zinc oxide, PZT, PVDF or other piezoelectric materials.

In a preferable embodiment, the triboelectric generator is a triboelectricity generating film; and/or the piezoelectric generator is a piezoelectricity generating film. The triboelectricity generating film and/or the piezoelectricity generating film therein can be a triboelectricity generating film and/or a piezoelectricity generating film in the prior art, which can be selected by one skilled in the art according to actual necessities and will not be limited here.

In another preferable embodiment, the triboelectric generator comprises a friction part-based knitted triboelectric generator in the prior art. For better understanding by one skilled in the art of the friction part-based knitted triboelectric generator comprised in the triboelectric generator, specific structure of the friction part-based knitted triboelectric generator comprised in the triboelectric generator in the prior art will be introduced in detail.

The friction part-based knitted triboelectric generator comprised in the triboelectric generator comprises: at least one first friction part and at least one second friction part; wherein: the at least one first friction part and the at least one second friction part are knitted with each other so as to form the friction part-based knitted triboelectric generator, and the at least one first friction part and/or the at least one second friction part comprise a friction interface capable of contacting friction; and the at least one first friction part and/or the at least one second friction part have an electrical signal output end of the friction part-based knitted triboelectric generator.

In a first embodiment, the friction part-based knitted triboelectric generator comprised in the triboelectric generator comprises: at least one first friction part and at least one second friction part. Wherein: the at least one first friction part comprises a first polymer insulator; the at least one second friction part comprises a second electric conductor; the first polymer insulator and the second electric conductor are knitted with each other so as to form the friction part-based knitted triboelectric generator; a friction interface is formed or friction interfaces are formed through contacting friction between the first polymer insulator and the second electric conductor, and/or between the first polymer insulator and the object in need of hair growing, and/or between the second electric conductor and the object in need of hair growing; and the second electric conductor serves as an electrical signal output end of the friction part-based knitted triboelectric generator.

In a second embodiment, the friction part-based knitted triboelectric generator comprised in the triboelectric generator comprises: at least one first friction part and at least one second friction part. Wherein: the at least one first friction part comprises a first electric conductor and a first polymer insulator; the at least one second friction part comprises a second electric conductor; the first polymer insulator is provided on an outside surface of the first electric conductor; the first electric conductor and the first polymer insulator are knitted with the second electric conductor so as to form the friction part-based knitted triboelectric generator; a friction interface is formed or friction interfaces are formed through contacting friction between the first electric conductor and the first polymer insulator, and/or between the first electric conductor and the second electric conductor, and/or between the first electric conductor and the object in need of hair growing, and/or between the first polymer insulator and the second electric conductor, and/or between the first polymer insulator and the object in need of hair growing, and/or between the second electric conductor and the object in need of hair growing; and the first electric conductor and/or the second electric conductor serve as an electrical signal output end of the friction part-based knitted triboelectric generator.

In a third embodiment, the friction part-based knitted triboelectric generator comprised in the triboelectric generator comprises: at least one first friction part and at least one second friction part. Wherein: the at least one first friction part comprises a first electric conductor and a first polymer insulator; the at least one second friction part comprises a second electric conductor and a second polymer insulator; the first polymer insulator is provided on an outside surface of the first electric conductor, and the second polymer insulator is provided on an outside surface of the second electric conductor; the first electric conductor and the first polymer insulator are knitted with the second electric conductor and the second polymer insulator so as to form the friction part-based knitted triboelectric generator; a friction interface is formed or friction interfaces are formed through contacting friction between the first electric conductor and the first polymer insulator, and/or between the first electric conductor and the second polymer insulator, and/or between the first electric conductor and the object in need of hair growing, and/or between the first electric conductor and the second electric conductor, and/or between the first polymer insulator and the second polymer insulator, and/or between the first polymer insulator and the object in need of hair growing, and/or between the first polymer insulator and the second electric conductor, and/or between the second polymer insulator and the object in need of hair growing; and/or between the second electric conductor and the second polymer insulator, and/or between the second electric conductor and the object in need of hair growing; and the first electric conductor and/or the second electric conductor serve as an electrical signal output end of the friction part-based knitted triboelectric generator.

In a fourth embodiment, the friction part-based knitted triboelectric generator comprised in the triboelectric generator comprises: at least one first friction part and at least one second friction part. Wherein: the at least one first friction part comprises a first electric conductor and a first polymer insulator; the at least one second friction part comprises a second polymer insulator; the first polymer insulator is provided on an outside surface of the first electric conductor; the first electric conductor and the first polymer insulator are knitted with the second polymer insulator so as to form the friction part-based knitted triboelectric generator; a friction interface is formed or friction interfaces are formed through contacting friction between the first electric conductor and the first polymer insulator, and/or between the first electric conductor and the second polymer insulator, and/or between the first electric conductor and the object in need of hair growing, and/or between the first polymer insulator and the second polymer insulator, and/or between the first polymer insulator and the object in need of hair growing, and/or between the second polymer insulator and the object in need of hair growing; and the first electric conductor serves as an electrical signal output end of the friction part-based knitted triboelectric generator.

In a fifth embodiment, the friction part-based knitted triboelectric generator comprised in the triboelectric generator comprises: at least one first friction part and at least one second friction part. Wherein: the at least one first friction part comprises a first electric conductor; the at least one second friction part comprises a second electric conductor; the first electric conductor and the second electric conductor are knitted with each other so as to form the friction part-based knitted triboelectric generator; a friction interface is formed or friction interfaces are formed through contacting friction between the first electric conductor and the second electric conductor, and/or between the first electric conductor and the object in need of hair growing, and/or between the second electric conductor and the object in need of hair growing; and the first electric conductor and/or the second electric conductor serve as an electrical signal output end of the friction part-based knitted triboelectric generator.

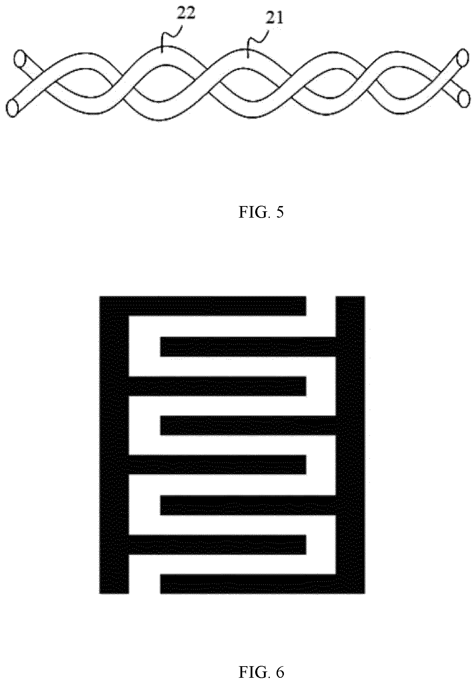

Further, in the afore-mentioned second, third, and fourth embodiments, the first polymer insulator may be provided on the outside surface of the first electric conductor by means of coating or winding and knitting, so as to form the at least one first friction part; specifically, FIG. 4 shows that a first polymer insulating layer 22 is provided on the outside surface of the first electric conductor 21 by means of coating so as to form the at least one first friction part, and FIG. 5 shows that the first polymer insulator 22 is provided on the outside surface of the first electric conductor 21 by means of winding and knitting so as to form the at least one first friction part. In addition, in the afore-mentioned third embodiment, the second polymer insulator may also be provided on the outside surface of the second electric conductor by means of coating or winding and knitting, so as to form the at least one second friction part. In other words, the first polymer insulator is provided on the outside surface of the first electric conductor by means of coating or winding and knitting, so as to form the at least one first friction part; and/or, the second polymer insulator is provided on the outside surface of the second electric conductor by means of coating or winding and knitting, so as to form the at least one second friction part. These can be selected by one skilled in the art according to actual necessities, and will not be limited here.

It should be noted that when the at least one first friction part is formed by means of winding and knitting, the at least one first friction part can be formed by winding and knitting of one first electric conductor 21 with one first polymer insulator 22, as shown in FIG. 5; the at least one first friction part can also be formed by winding and knitting of one first electric conductor with multiple first polymer insulators; the at least one first friction part can also be formed by winding and knitting of multiple first electric conductors with one first polymer insulator; and the at least one first friction part can even be formed by winding and knitting of multiple first electric conductors with multiple first polymer insulators; these can be selected by one skilled in the art according to actual necessities and will not be limited here. For description about forming the at least one second friction part by means of winding and knitting, please refer to the description about forming the at least one first friction part by means of winding and knitting, which will not be repeated here.

Further, in the first embodiment, the first polymer insulator may comprise a plurality of polymer insulated wires forming the first polymer insulator by means of winding and knitting, and/or the second electric conductor may comprise a plurality of conductive wires forming the second electric conductor by means of winding and knitting; in the second embodiment, the first electric conductor may comprise a plurality of conductive wires forming the first electric conductor by means of winding and knitting, and/or the first polymer insulator may comprise a plurality of polymer insulated wires forming the first polymer insulator by means of winding and knitting, and/or the second electric conductor may comprise a plurality of conductive wires forming the second electric conductor by means of winding and knitting; in the third embodiment, the first electric conductor may comprise a plurality of conductive wires forming the first electric conductor by means of winding and knitting, and/or the first polymer insulator may comprise a plurality of polymer insulated wires forming the first polymer insulator by means of winding and knitting, and/or the second electric conductor may comprise a plurality of conductive wires forming the second electric conductor by means of winding and knitting, and/or the second polymer insulator may comprise a plurality of polymer insulated wires forming the second polymer insulator by means of winding and knitting; in the fourth embodiment, the first electric conductor may comprise a plurality of conductive wires forming the first electric conductor by means of winding and knitting, and/or the first polymer insulator may comprise a plurality of polymer insulated wires forming the first polymer insulator by means of winding and knitting, and/or the second polymer insulator may comprise a plurality of polymer insulated wires forming the second polymer insulator by means of winding and knitting; in the fifth embodiment, the first electric conductor may comprise a plurality of conductive wires forming the first electric conductor by means of winding and knitting, and/or the second electric conductor may comprise a plurality of conductive wires forming the second electric conductor by means of winding and knitting; these can be selected by one skilled in the art according to actual necessities, and will not be limited here.

Afore-mentioned means of forming the first friction part, the second friction part, the first electric conductor, the first polymer insulator, the second electric conductor and the second polymer insulator can be used in any way of combination, which can be selected by one skilled in the art according to actual necessities and will not be limited here. For example, in the fourth embodiment, the first polymer insulator is formed through winding and knitting of a plurality of polymer insulated wires, and the first polymer insulator coats the outside surface of the first electric conductor, thereby forming the at least one first friction part; the second polymer insulator is formed through winding and knitting of a plurality of polymer insulated wires, thereby forming the at least one second friction part; and the friction part-based knitted triboelectric generator is formed through winding and knitting of the at least one first friction part and the at least one second friction part.

Further, for better contacting friction of the surface constituting the friction interface in the friction part-based knitted triboelectric generator, a bulge structure may be provided on at least one surface constituting the friction interface in the friction part-based knitted triboelectric generator. The types and quantities of the dips and bulges comprised in the bulge structure are not limited in the present application, and can be flexibly provided by one skilled in the art.

In Example 1, materials for the first electric conductor and the second electric conductor are indium tin oxide, graphene, silver nanowire film, metal or alloy; wherein: the metal is gold, silver, platinum, palladium, aluminum, nickel, copper, titanium, chromium, selenium, iron, manganese, molybdenum, tungsten or vanadium; and the alloy is aluminum alloy, titanium alloy, magnesium alloy, beryllium alloy, copper alloy, zinc alloy, manganese alloy, nickel alloy, lead alloy, tin alloy, cadmium alloy, bismuth alloy, indium alloy, gallium alloy, tungsten alloy, molybdenum alloy, niobium alloy or tantalum alloy.

In Example 1, the material for the first polymer insulator and the second polymer insulator is any selected from polytetrafluoroethylene, polydimethylsiloxane, polyimide, aniline formaldehyde resin, polyoxymethylene, ethyl cellulose, polyamide, melamine formaldehyde, polyethylene glycol succinate, cellulose, cellulose acetate, polyethylene adipate, poly(diallyl phthalate), fiber (recycled) sponge, polyurethane elastomer, styrene propylene copolymer, styrene-butadiene copolymer, artificial fiber, polymethyl, methacrylate, polyvinyl alcohol, polyester, polyisobutylene, polyurethane flexible sponges, polyethylene terephthalate, polyvinyl butyral, formaldehyde phenol, neoprene, butadiene propylene copolymer, natural rubber, polyacrylonitrile, acrylonitrile vinyl chloride and polyethylene bisphenol carbonates.

In order to improve the effect of triboelectricity generating, it is preferable that different polymer materials are used for the first polymer material surface and the second polymer material surface, which are capable of contacting friction and are comprised in the at least one first friction part and/or the at least one second friction part. For example: in the third and fourth embodiments, since contacting friction may occur between the first polymer insulator and the second polymer insulator, it is preferable that different polymer materials are used for the first polymer insulator and the second polymer insulator, so as to improve the effect of triboelectricity generating.

It should be understood that the friction part-based knitted triboelectric generator is provided on the object in need of hair growing when it is used in the field of electrical stimulation hair growing, and the object in need of hair growing can be replaced with other objects by one skilled in the art when the friction part-based triboelectric generator is used in other fields; this will not be limited here.

To avoid electromagnetic interference from external environment and avoid the influence of external environmental factors (such as temperature, humidity and/or dust, etc.), it is preferable that a triboelectric generator provided with a shielding layer and a protective layer in sequence at outside, and/or a piezoelectric generator, and/or a pressure inductive cable based on a triboelectric generator and/or a piezoelectric generator, and/or a piezoelectric and triboelectric hybrid generator are adopted in the electrical pulse module 20, thereby ensuring normal operation of the electrical pulse module 20.

To prevent the normal operation of the electrical pulse module 20 from being affected by merely the electromagnetic interference of external environment, a triboelectric generator provided with a shielding layer at outside, and/or a piezoelectric generator, and/or a pressure inductive cable based on a triboelectric generator and/or a piezoelectric generator, and/or a piezoelectric and triboelectric hybrid generator may be adopted in the electrical pulse module 20; to prevent the normal operation of the electrical pulse module 20 from being affected by external environmental factors (such as temperature, humidity and/or dust, etc.), a triboelectric generator provided with a protective layer at outside, and/or a piezoelectric generator, and/or a pressure inductive cable based on a triboelectric generator and/or a piezoelectric generator, and/or a piezoelectric and triboelectric hybrid generator may be adopted in the electrical pulse module 20.

To increase the user's comfort level, it is preferable that a triboelectric generator, and/or a piezoelectric generator, and/or a pressure inductive cable based on a triboelectric generator and/or a piezoelectric generator, and/or a piezoelectric and triboelectric hybrid generator, which are flexible, are adopted in the electrical pulse module 20.

Optionally, the number of the triboelectric generator, and/or the piezoelectric generator, and/or the pressure inductive cable based on a triboelectric generator and/or a piezoelectric generator, and/or the piezoelectric and triboelectric hybrid generator comprised in the electrical pulse module 20 may be one or multiple; this can be selected by one skilled in the art according to actual necessities and will not be limited here. When multiple triboelectric generators, and/or piezoelectric generators, and/or pressure inductive cables based on a triboelectric generator and/or a piezoelectric generator, and/or piezoelectric and triboelectric hybrid generators are adopted, the multiple triboelectric generators, and/or piezoelectric generators, and/or pressure inductive cables based on a triboelectric generator and/or a piezoelectric generator, and/or piezoelectric and triboelectric hybrid generators are connected in series or in parallel, and the multiple triboelectric generators, and/or piezoelectric generators, and/or pressure inductive cables based on a triboelectric generator and/or a piezoelectric generator, and/or piezoelectric and triboelectric hybrid generators can be provided by means of tiling and/or stacking; these will not be limited here, and can be selected by one skilled in the art according to actual necessities. Preferably, the multiple triboelectric generators, and/or piezoelectric generators, and/or pressure inductive cables based on a triboelectric generator and/or a piezoelectric generator, and/or piezoelectric and triboelectric hybrid generators are provided in tiled arrays.

The electrical stimulation module 30 comprises a plurality of electrodes, wherein the position and/or size of each electrode therein can be flexibly provided by one skilled in the art according to the position and/or size of the area in need of hair growing, and will not be limited here. However, it should be noted that there must be a potential difference among the electrodes pre-applied with electrical stimulation in the plurality of electrodes comprised in the electrical stimulation module 30, thereby ensuring that electrical pulses can be applied to the area in need of hair growing through the plurality of electrodes for electrical stimulation.

To improve the effect of electrical stimulation, at least one electrical stimulation bulge is provided on the surface at one side of the plurality of electrodes in contact with the area in need of hair growing; wherein the number, size, shape, providing and/or shape formed after providing of the at least one electrical stimulation bulge can be selected by one skilled in the art according to actual necessities, and will not be limited here. Preferably, the plurality of electrical stimulation bulges is provided on the surface at one side of the plurality of electrodes in contact with the area in need of hair growing, and the plurality of electrical stimulation bulges is provided in a circular array. More preferably, a plurality of electrical stimulation bulges matching the size of hair follicles of the object in need of hair growing is provided on the surface at one side of the plurality of electrodes in contact with the area in need of hair growing, and the plurality of electrical stimulation bulges is provided in a circular array. These two preferable ways of providing are more suitable for the distribution and size of hair follicles of the object in need of hair growing, so that the electrical pulses can be better applied to the area in need of area growing for electrical stimulation.

The plurality of electrodes may be strip-shaped electrodes or interdigitated electrodes as shown in FIG. 6; of course, they may also be in other shapes, which will not be limited here. In addition, the electrical stimulation module 30 may be in direct or indirect contact with the area in need of hair growing, which will not be limited here.

For better understanding of the connection mode of the electrical pulse module 20 and the electrical stimulation module 30 in Example 1 by one skilled in the art, connection modes of the electrical pulse module 20 and the electrical stimulation module 30 will be described in detail below.

When the electrical pulse module 20 comprises two output ends between which there is a potential difference, and the electrical stimulation module 30 comprises two electrodes, the two output ends comprised in the electrical pulse module 20 can be respectively and correspondingly connected to the two electrodes comprised in the electrical stimulation module 30; it is also possible that either of the two output ends comprised in the electrical pulse module 20 is selected to be connected to either of the two electrodes comprised in the electrical stimulation module 30, while the other electrode comprised in the electrical stimulation module 30 is directly connected to the object in need of hair growing or other reference potential points.

When the electrical pulse module 20 comprises two output ends between which there is no potential difference (namely, the two output ends are conducted or the potentials thereof are the same), and the electrical stimulation module 30 comprises two electrodes, either of the two output ends comprised in the electrical pulse module 20 may be selected to be connected to either of the two electrodes comprised in the electrical stimulation module 30, while the other electrode comprised in the electrical stimulation module 30 is directly connected to the object in need of hair growing or other reference potential points.

When the electrical pulse module 20 comprises one output end and the electrical stimulation module 30 comprises two electrodes, the one output end comprised in the electrical pulse module 20 may be connected to either of the two electrodes comprised in the electrical stimulation module 30, while the other electrode comprised in the electrical stimulation module 30 is directly connected to the object in need of hair growing or other reference potential points. That is to say, regardless of the connection mode, it must make sure that a potential difference can be generated between the electrodes comprised in the electrical stimulation module 30 for pre-applied electrical stimulation. Cases are similar for other situations and will not be repeated here.

In Example 1, when the electrical pulse module 20 comprises a friction part-based knitted triboelectric generator or a pressure inductive cable based on a triboelectric generator and/or a piezoelectric generator, the at least one first friction part and the at least one second friction part can be used after being knitted by using an existing knitting method into a friction part-based knitted triboelectric generator having a predetermined shape, or the pressure inductive cable based on a triboelectric generator and/or a piezoelectric generator can be used after being knitted into a predetermined shape. For example, one first friction part and one second friction part are knitted by plain knitting, or two pressure inductive cables based on a triboelectric generator and/or a piezoelectric generator are knitted by plain knitting, so as to form an electrical pulse module 20 having a hat or cap shape, which can be used after being provided at an area in need of hair growing on the head of an object in need of hair growing; and the electrical stimulation module 30 is provided on an inside surface of the electrical pulse module 20 close to the object in need of hair growing.

The predetermined shape referred to in Example 1 can be any shape, for example, it can be a hat or cap shape, and can be a headgear shape, a turban shape or a collar shape, and can also be a sheet shape, etc.; this can be selected by one skilled in the art according to actual necessities, and will not be limited here.

Of course, after the at least one first friction part and the at least one second friction part are knitted into a predetermined shape and/or after the pressure inductive cable based on a triboelectric generator and/or a piezoelectric generator is knitted into a predetermined shape, they can also be used in combination with any one or more of other generators referred to in Example 1; this will not be limited here, and can be selected by one skilled in the art according to actual necessities.

Optionally, a protective layer (not shown) of the hair growing device is further provided outside the vibration module 10, the electrical pulse module 20 and/or the electrical stimulation module 30, and is used for protecting the electrical pulse module 20 and/or the electrical stimulation module 30.

Wherein: the material used for the protective layer of the hair growing device is selected from any of polytetrafluoroethylene, polydimethylsiloxane, polyimide, aniline formaldehyde resin, polyoxymethylene, ethyl cellulose, polyamide, melamine formaldehyde, polyethylene glycol succinate, cellulose, cellulose acetate, polyethylene adipate, poly(diallyl phthalate), fiber (recycled) sponge, polyurethane elastomer, styrene propylene copolymer, styrene-butadiene copolymer, artificial fiber, polymethyl, methacrylate, polyvinyl alcohol, polyester, polyisobutylene, polyurethane flexible sponges, polyethylene terephthalate, polyvinyl butyral, formaldehyde phenol, neoprene, butadiene propylene copolymer, natural rubber, polyacrylonitrile, acrylonitrile vinyl chloride and polyethylene bisphenol carbonates.

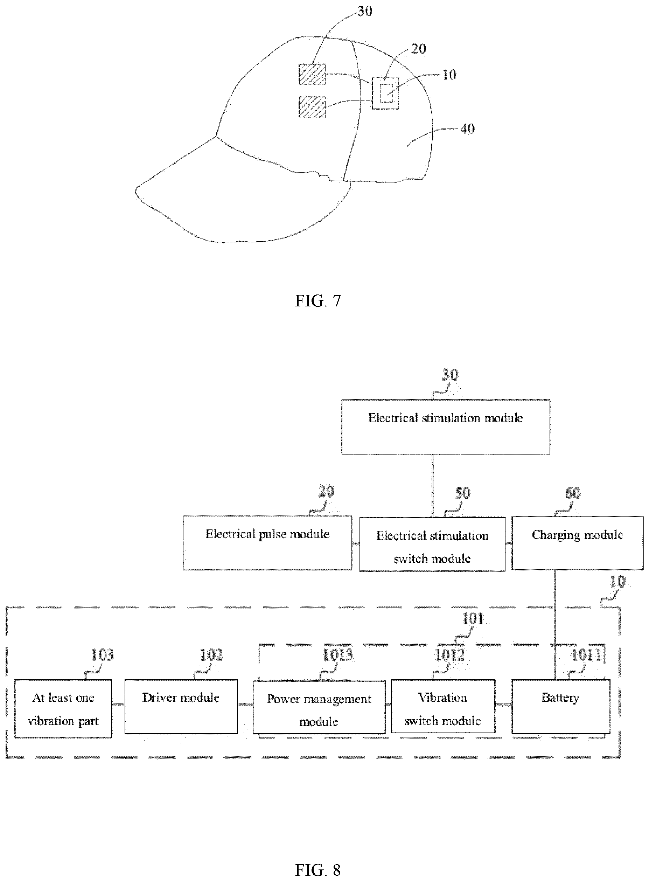

In addition, as shown in FIG. 7, the electrical stimulation hair growing device in Example 1 further comprises: a wearable body 40. Wherein: the wearable body 40 is worn on the object in need of hair growing (not shown), and is provided with the vibration module 10, the electrical pulse module 20 and/or the electrical stimulation module 30. When the wearable body 40 is worn on the object in need of hair growing, the vibration generated by the vibration module 10 and/or the mechanical energy from external environment are acted on the object in need of hair growing, reciprocating extrusion or loosening will occur between the electrical pulse module 20 and the object in need of hair growing, and relative motion will be generated due to relative friction, wherein relative motion is converted into electrical pulses before it is output.

When the electrical stimulation hair growing device in Example 1 comprises the wearable body 40, the vibration module 10, the electrical pulse module 20 and/or the electrical stimulation module 30 can be provided on the inside surface, the outside surface and/or the interlayer of the wearable body, and this can be selected by one skilled in the art according to actual necessities and will not be limited here; besides, the wearable body 40 is a wearable object in the prior art, such as: a hat or cap, a headgear, a turban, a collar, or a sheet, etc., which can be selected by one skilled in the art according to actual necessities and will not be limited here. Specifically, the wearable body 40 in FIG. 7 is a cap, and the vibration module 10, the electrical pulse module 20 and the electrical stimulation module 30 are respectively provided on the inside surface of the cap top.

In Example 1, an electrical stimulation switch module (not shown) may be further provided between the electrical pulse module 20 and the electrical stimulation module 30, and is used for controlling whether the electrical pulse module 20 outputs electrical pulses to the electrical stimulation module 30. In other words, the electrical stimulation switch module is respectively connected to the electrical pulse module 20 and the electrical stimulation module 30. When the electrical stimulation switch module is turned on, the electrical pulse module 20 and the electrical stimulation module 30 are conducted through the electrical stimulation switch module, and the electrical pulse module 20 outputs electrical pulses to the electrical stimulation module 30 for electrical stimulation; when the electrical stimulation switch module is turned off, the electrical stimulation switch module disconnects the electrical pulse module 20 from the electrical stimulation module 30, and the electrical pulse module 20 stops outputting electrical pulses to the electrical stimulation module 30 for electrical stimulation.

The electrical stimulation switch module can be a switch in the prior art. For example, the electrical stimulation switch module can be a key switch, a touch switch or a film switch, and the type thereof can be selected by one skilled in the art according to actual necessities and will not be limited here. Since film switches are light, thin and flexible, which can increase the comfort level of use, the electrical stimulation switch module is preferably a film switch.

The electrical stimulation hair growing device provided in Example 1 generates vibration with a predetermined frequency and a predetermined amplitude through the vibration module, and accurately controls the electrical pulses output by the electrical pulse module to the electrical stimulation module, so as to accurately apply different electrical pulses to different objects in need of hair growing, thereby activating epidermal hair follicles and promoting hair growth; besides, since a triboelectric generator, and/or a piezoelectric generator, and/or a pressure inductive cable based on a triboelectric generator and/or a piezoelectric generator, and/or a piezoelectric and triboelectric hybrid generator are adopted in the electrical pulse module, it is possible to directly convert the mechanical energy in external environment into electrical pulses when there is no or insufficient external power supply, so that electrical stimulation can be performed by the electrical stimulation module, which is energy-efficient and environmentally friendly, and can also avoid the failure to use the electrical stimulation hair growing device caused by the absence of external power supply; moreover, the electrical stimulation hair growing device provided in Example 1 is simple in structure and manufacturing process, and has low cost, so it is suitable for large-scale industrial production.

FIG. 8 is a diagram showing a circuit module in Example 2 for the electrical stimulation hair growing device provided in the present disclosure. As shown in FIG. 8, the difference between the electrical stimulation hair growing device in Example 2 and that in Example 1 is that the electrical stimulation hair growing device in Example 2 further comprises: an electrical stimulation switch module 50 and a charging module 60; wherein: the electrical stimulation switch module 50 is respectively connected to the electrical pulse module 20 and the electrical stimulation module 30, and is used for enabling the electrical pulse module 20 to output electrical pulses to the electrical stimulation module 30 when the electrical pulse module 20 and the electrical stimulation module 30 are conducted by the electrical stimulation switch module 50; the charging module 60 is respectively connected to the electrical stimulation switch module 50 and the power module 101, and is used for preprocessing the electrical pulses output by the electrical pulse module 20 when the electrical pulse module 20 and the charging module 60 are conducted by the electrical stimulation switch module 50, and for outputting the preprocessed electrical pulses to the power module 101 for charging.

Wherein: when the power module 101 comprises the battery 1011, the vibration switch module 1012 and the power management module 1013, the charging module 60 is respectively connected to the electrical stimulation switch module 50 and the battery 1011, and is used for preprocessing the electrical pulses output by the electrical pulse module 20 when the electrical pulse module 20 and the charging module 60 are conducted by the electrical stimulation switch module 50, and for outputting the preprocessed electrical pulses to the battery 1011 for charging.

Optionally, the charging module 60 comprises a rectification module (not shown), an amplification module (not shown) and a filtration module (not shown); wherein: the rectification module is connected to the electrical stimulation switch module 50, and is used for rectifying the electrical pulses output by the electrical pulse module 20 through the electrical stimulation switch module 50; the amplification module is connected to the rectification module, and is used for amplifying the electrical signals output by the rectification module; and the filtration module is respectively connected to the amplification module and the power module 101, and is used for filtering out interference noise in the electrical signal output by the amplification module, so that the power module 101 is charged.

Specifically, when the power module 101 comprises the battery 1011, the vibration switch module 1012 and the power management module 1013, the charging module 60 comprises a rectification module, an amplification module and a filtration module; wherein: the rectification module is connected to the electrical stimulation switch module 50, and is used for rectifying the electrical pulses output by the electrical pulse module 20 through the electrical stimulation switch module 50; the amplification module is connected to the rectification module, and is used for amplifying the electrical signals output by the rectification module; and the filtration module is respectively connected to the amplification module and the battery 1011 in the power module 101, and is used for filtering out interference noise in the electrical signal output by the amplification module, so that the battery 1011 in the power module 101 is charged.

It should be noted that the rectification module, the amplification module and the filtration module comprised in the charging module 60 are all optional modules, which can be selected by one skilled in the art according to actual necessities and will not be limited here. For example, the amplification module can be omitted if amplification is not required. In addition, the rectification module, the amplification module and the filtration module comprised in the charging module 60 all adopt rectification modules, amplification modules and filtration modules in the prior art, which will not be limited here.

In the second embodiment, the electrical stimulation switch module 50 is respectively connected to the electrical pulse module 20, the electrical stimulation module 30 and the charging module 60. When the electrical stimulation switch module 50 is turned on, the electrical pulse module 20 and the stimulation module 30 are conducted through the electrical stimulation switch module 50, and the electrical pulse module 20 outputs electrical pulses to the electrical stimulation module 30 for electrical stimulation; and, when the electrical stimulation switch module 50 is turned off, the electrical stimulation switch module 50 conducts the electrical pulse module 20 and the charging module 60, and the electrical pulses output by the electrical pulse module 20 are output to the power module 101 after being preprocessed by the charging module 60, so that the power module 101 is charged.

More specifically, when the power module 101 comprises the battery 1011, the vibration switch module 1012 and the power management module 1013, the electrical stimulation switch module 50 is respectively connected to the electrical pulse module 20, the electrical stimulation module 30 and the charging module 60. When the electrical stimulation switch module 50 is turned on, the electrical pulse module 20 and the stimulation module 30 are conducted through the electrical stimulation switch module 50, and the electrical pulse module 20 outputs electrical pulses to the electrical stimulation module 30 for electrical stimulation; and, when the electrical stimulation switch module 50 is turned off, the electrical stimulation switch module 50 conducts the electrical pulse module 20 and the charging module 60, and the electrical pulses output by the electrical pulse module 20 are output to the battery 1011 after being preprocessed by the charging module 60, so that the battery 1011 is charged.

Wherein: the electrical stimulation switch module 50 can be a switch in the prior art, for example, the electrical stimulation switch module 50 can be a single-pole double-throw key switch or the like; the type thereof can be selected by one skilled in the art according to actual necessities and will not be limited here.

In Example 2, the electrical pulse module 20 charges the battery 1011 in the power module 101 through the charging module 60, so the battery 1011 adopts a rechargeable nickel-cadmium battery, nickel-metal hydride battery, lithium-ion battery, or lead storage battery in the prior art, and will not be limited here.

Except for the afore-mentioned differences, please refer to the description of the electrical stimulation hair growing device in Example 1 for other description of the electrical stimulation hair growing device in Example 2, which will not be repeated here.

The electrical stimulation hair growing device provided in Example 2 generates vibration with a predetermined frequency and a predetermined amplitude through the vibration module, and accurately controls the electrical pulses output by the electrical pulse module to the electrical stimulation module, so as to accurately apply different electrical pulses to different objects in need of hair growing, thereby activating epidermal hair follicles and promoting hair growth; besides, since a triboelectric generator, and/or a piezoelectric generator, and/or a pressure inductive cable based on a triboelectric generator and/or a piezoelectric generator, and/or a piezoelectric and triboelectric hybrid generator are adopted in the electrical pulse module, it is possible to directly convert the mechanical energy in external environment into electrical pulses when there is no or insufficient external power supply, so that electrical stimulation can be performed by the electrical stimulation module, which is energy-efficient and environmentally friendly, and can also avoid the failure to use the electrical stimulation hair growing device caused by the absence of external power supply; meanwhile, the electrical stimulation hair growing device provided in the present disclosure is light and small, and is easy for users to carry and/or use; besides, the electrical stimulation hair growing device provided in Example 2 can convert the mechanical energy in external environment into electric energy by means of the electrical pulse module, so as to charge the power module, which can prolong the power supply time of the power module, as well as is energy-efficient and environmentally friendly; moreover, it is simple in structure and manufacturing process, and has low cost, so it is suitable for large-scale industrial production.

Both the electrical stimulation hair growing device in Example 1 and that in Example 2 of the present disclosure can be detachable and/or portable. In this way, it is convenient for the user to carry and/or use, thereby ensuring that the electrical stimulation hair growing device of the present disclosure can be used by the user in most occasions.

Afore-mentioned are merely embodiments of the present disclosure. Taught by the present disclosure, one skilled in the art can make other improvements or modifications on the basis of the above-mentioned examples. It should be understood by one skilled in the art that the afore-mentioned illustration is merely for better explanation of the present disclosure, and the scope of protection of the present disclosure should follow the scope of protection of claims.

## Claims

1. An electrical stimulation hair growing device, comprising: a vibration module, an electrical pulse module and an electrical stimulation module; wherein:
the vibration module is used for generating vibration with a predetermined frequency and a predetermined amplitude;
the electrical pulse module is provided in contact with the vibration module, and is used for converting vibration acted thereon by the vibration module into electrical pulses and outputting the electrical pulses, and/or for converting mechanical energy acted thereon by external environment into electrical pulses and outputting the electrical pulses; and
the electrical stimulation module is provided on an area in need of hair growing, is connected to the electrical pulse module and is used for acting the electrical pulses output by the electrical pulse module on the area in need of hair growing for electrical stimulation.

2. The electrical stimulation hair growing device according to claim 1, wherein the vibration module further comprises: a power module, a driver module, and at least one vibration part;
the power module being connected to the driver module, and being used for providing electric energy to the driver module;
the driver module being connected to the at least one vibration part, and being used for controlling the at least one vibration part to vibrate at the predetermined frequency and the predetermined amplitude; and
the at least one vibration part being provided in contact with the electrical pulse module, and being used for generating vibration having the predetermined frequency and the predetermined amplitude so as to act the vibration on the electrical pulse module.

3. The electrical stimulation hair growing device according to claim 2, wherein the power module further comprises a battery, a vibration switch module and a power management module;
the battery being connected to the vibration switch module, and being used for providing electric energy to the power management module through the vibration switch module;
the vibration switch module being connected to the power management module, and being used for controlling whether the battery provides electric energy to the power management module; and
the power management module being connected to the driver module, and being used for converting the electric energy output by the battery through the vibration switch module, so as to provide electric energy to the driver module.

4. The electrical stimulation hair growing device according to claim 1, wherein the electrical stimulation hair growing device further comprises an electrical stimulation switch module; the electrical stimulation switch module being respectively connected to the electrical pulse module and the electrical stimulation module, and being used for enabling the electrical pulse module to output electrical pulses to the electrical stimulation module when the electrical pulse module and the electrical stimulation module are conducted by the electrical stimulation switch module.

5. The electrical stimulation hair growing device according to claim 2 or 3, wherein the electrical stimulation hair growing device further comprises an electrical stimulation switch module and a charging module; the electrical stimulation switch module being respectively connected to the electrical pulse module and the electrical stimulation module, and being used for enabling the electrical pulse module to output electrical pulses to the electrical stimulation module when the electrical pulse module and the electrical stimulation module are conducted by the electrical stimulation switch module; and the charging module being respectively connected to the electrical stimulation switch module and the power module, and being used for preprocessing the electrical pulses output by the electrical pulse module when the electrical pulse module and the charging module are conducted by the electrical stimulation switch module, and for outputting the preprocessed electrical pulses to the power module for charging.

6. The electrical stimulation hair growing device according to claim 2, wherein a transmission insulating part for transmitting vibration and for avoiding conduction of the at least one vibration part and the electrical pulse module is further provided between the at least one vibration part and the electrical pulse module.

7. The electrical stimulation hair growing device according to claim 2 or 6, wherein the vibration part is a rotor motor, a longitudinal linear motor or a transverse linear motor.

8. The electrical stimulation hair growing device according to claim 7, wherein: the number of vibration parts is multiple, the multiple vibration parts comprise the same number of the longitudinal linear motors and the transverse linear motors, and the longitudinal linear motors and the transverse linear motors are uniformly distributed in arrays on the electrical pulse module.

9. The electrical stimulation hair growing device according to claim 1, wherein: the electrical stimulation module further comprises a plurality of electrodes; and at least one electrical stimulation bulge is provided on a side surface where the plurality of electrodes is in contact with the area in need of hair growing.

10. The electrical stimulation hair growing device according to claim 1 or 9, wherein the electrical pulse module comprises: a triboelectric generator, and/or a piezoelectric generator, and/or a pressure inductive cable based on a triboelectric generator and/or a piezoelectric generator, and/or a piezoelectric and triboelectric hybrid generator.

11. The electrical stimulation hair growing device according to claim 10, wherein: the triboelectric generator is a triboelectricity generating film; and/or the piezoelectric generator is a piezoelectricity generating film.

12. The electrical stimulation hair growing device according to claim 10, wherein the triboelectric generator comprises a friction part-based knitted triboelectric generator;
the friction part-based knitted triboelectric generator comprising: at least one first friction part and at least one second friction part; wherein:
the at least one first friction part and the at least one second friction part are knitted with each other to form the friction part-based knitted triboelectric generator, and at least one of the at least one first friction part and the at least one second friction part comprises a friction interface capable of contacting friction; and
the at least one first friction part and/or the at least one second friction part have an electrical signal output end of the friction part-based knitted triboelectric generator.

13. The electrical stimulation hair growing device according to claim 10, wherein the pressure inductive cable based on a triboelectric generator and/or a piezoelectric generator is knitted to form the electrical pulse module having a predetermined shape.

14. The electrical stimulation hair growing device according to claim 12, wherein the at least one first friction part and the at least one second friction part are knitted to form the electrical pulse module having a predetermined shape.

15. The electrical stimulation hair growing device according to claim 13 or 14, wherein the predetermined shape is a hat or cap shape, a headgear shape, a turban shape, a collar shape or a sheet shape.

16. The electrical stimulation hair growing device according to claim 1, wherein the electrical stimulation hair growing device further comprises a wearable body; the wearable body being worn on an object in needed of hair growing, and being provided with the at least one vibration module, the electrical pulse module and/or the electrical stimulation module.

17. The electrical stimulation hair growing device according to claim 16, wherein the wearable body is a hat or cap, a headgear, a turban, a collar or a sheet.

18. The electrical stimulation hair growing device according to claim 1, wherein the electrical stimulation hair growing device is a detachable and/or portable electrical stimulation hair growing device.
